(19) Europäisches Patentamt
European Patent Office
Office européen des brevets

(11) Publication number: **0 045 661 B1**

(12) # EUROPEAN PATENT SPECIFICATION

(45) Date of publication of patent specification: **12.10.83**

(51) Int. Cl.³: **A 61 K 31/557**

(21) Application number: **81303564.9**

(22) Date of filing: **05.08.81**

(54) **Pharmaceutical compositions comprising prostaglandins.**

(30) Priority: **06.08.80 US 175467**

(43) Date of publication of application:
**10.02.82 Bulletin 82/6**

(45) Publication of the grant of the patent:
**12.10.83 Bulletin 83/41**

(84) Designated Contracting States:
**BE CH DE FR GB IT LI NL SE**

(56) References cited:
**DE - A - 2 719 901**
**US - A - 3 978 229**

(73) Proprietor: **THE UPJOHN COMPANY**
**301 Henrietta Street**
**Kalamazoo, Michigan 49001 (US)**

(72) Inventor: **Wilks, John William**
**6728 Trotwood Street**
**Portage Michigan (US)**

(74) Representative: **Perry, Robert Edward et al,**
**GILL JENNINGS & EVERY 53-64 Chancery Lane**
**London WC2A 1HN (GB)**

EP 0 045 661 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Pharmaceutical compositions comprising prostaglandins

This invention relates to compositions comprising a combination of prostaglandin compounds which may act synergically to induce menses and interrupt early pregnancy in female primates, particularly humans.

Prostaglandin compounds of the $F_\alpha$ series have been disclosed as being useful in stimulating smooth muscle, inhibiting gastric secretion, decongesting nasal passages, decreasing blood platelet adhesion, and for a wide variety of purposes in reproductive medicine, e.g. labour induction, abortion, cervical dilation, regulation of estrus and regulation of the menstrual cycle. Prostaglandin compounds of the E series have been disclosed as being useful in stimulating smooth muscle, affecting lipolytic activity, inhibiting gastric secretion, controlling spasms and facilitating breathing in asthmatic conditions, decongesting nasal passages, decreasing blood platelet adhesion, and for a wide variety of uses in reproductive medicine, e.g. labour induction, abortion, cervical dilation, regulation of the estrus and regulation of the menstrual cycles.

While a number of prostaglandins have been shown to be effective as luteolytic agents in various animal tests, it is difficult to find a prostaglandin which is 100% effective in inducing menses in female primates but which exhibits little or no toxicity or side effects. "Luteolytic" agents are those which cause corpus luteum regression. A functional corpus luteum is essential in early pregnancy.

U.S. Patent Specification No. 3,978,229, discloses the luteolytic effect of a combination of $PGF_{2\alpha}$ and $PGE_2$ in female hamsters, and a test for the anti-nidatory effect of a combination of 16,16-dimethyl-$PGE_2$ and 15(S), 16(R)-dimethyl-$PGF_{2\alpha}$, in female rats. However, such disclosures are of limited value in assessing the existence of primate luteolytic activity, since it is not possible to predict accurately the luteolytic activity of a prostaglandin combination in primates using rodent data. See, for example, "The Use of PG's in Human Reproduction", *Population Reports,* Population Information Program, The Johns Hopkins University, Prostaglandins, Series G, No. 8 (March 1980); and J. W. Wilks, "A Procedure for Evaluating Luteolytic Agents in Primates", *Ovarian Follicular and Corpus Luteum Function,* C. P. Channing *et al.,* Eds., pp. 757—766 (Plenum Press, New York 1979).

Various control mechanisms exist governing corpus luteum function in mammalian species. The uterus apparently regulates corpus luteum function in infraprimate animals, but the role of the uterus in primate luteal function has not been established. Thus, while $PGF_{2\alpha}$, a physiological luteolytic substance of uterine origin, has been successfully employed to regulate estrous cycles of domestic animals, J. W. Lauderdale *et al.,* J. Anim. Sci. 38:964 (1974), it is ineffective in controlling the human corpus luteum, W. J. LeMaire *et al,* Prostaglandins 1:259 (1972).

An effective luteolytic method of inducing menses in females must be able to counteract the corpus luteum-stimulating effects of chorionic gonadotropin. Agents which have been shown to be effective during non-fertile menstrual cycles have been ineffective during early gestation and in non-pregnant women given exogenous human chorionic gonadotropin (hCG). See, for example, J. W. Wilks, *supra,* and references cited therein.

A problem associated with known prostaglandin compositions for luteolysis is the acute toxicity of some of the prostaglandins. For example, 16,16-dimethyl-$PGE_2$ is part of the most potent luteolytic combination disclosed in US Patent Specification No. 3,978,229. The other prostaglandin is 15(S),16(R)-dimethyl-$PGF_{2\alpha}$. However, the "synergic" combination employed in the rat included 250 $\mu$g/kg body weight of 16,16-dimethyl $PGE_2$ which is very toxic in primates, causing convulsions and death at doses as low as 100 $\mu$g/kg body weight.

Known prostaglandins of the E and F series include 5-oxa-17-phenyl-18,19,20-trinor-$PGF_{1\alpha}$ and its alkyl esters, which are disclosed in US Patent Specification No. 3,864,387. 9-Deoxy-16,16-dimethyl-9-methylene-$PGE_2$ is disclosed in US Patent Specification No. 4,165,436. $PGF_{2\alpha}$ 1,15-lactone is disclosed in US Patent Specification No. 4,045,449. 5-Oxa-17-phenyl-18,19,20-trinor-$PGF_{1\alpha}$ amide is disclosed in U.S. Patent Specification No. 4,081,478.

According to the present invention, a composition comprises a first prostaglandin, of the formula

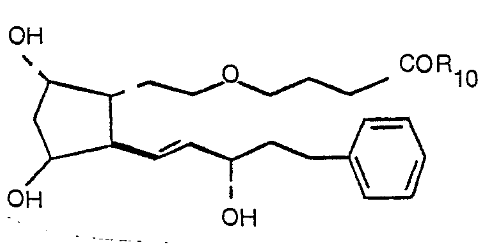

wherein $R_{10}$ is $NH_2$, OH, $C_{1-12}$ alkoxy or OM and M is a pharmacologically acceptable cation, and a second prostaglandin which is $PGF_{2\alpha}$, 1,15-lactone or is of the formula

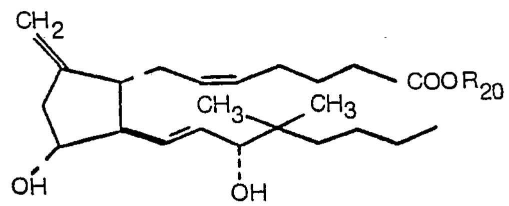

wherein $R_{20}$ is hydrogen, $C_{1-12}$ alkyl or a pharmacologically acceptable cation.

The first prostaglandin is the acid form, amide, alkyl ester or salt of 5-oxa-17-phenyl-18,19,20-trinor-$PGF_{1\alpha}$. The second prostaglandin is the acid form, or an alkyl ester or a salt, of 9-deoxo-16,16-dimethyl-9-methylene-$PGE_2$, or $PGF_{2\alpha}$, 1,15-lactone.

If an alkyl ester of the first or second prostaglandin is used, this may be a methyl, ethyl, propyl, butyl, pentyl, hexyl, heptyl, octyl, nonyl, decyl, undecyl or dodecyl ester, or any isomeric form thereof.

A composition of the present invention may be administered to a female primate, and is of particular value when administered to a female human, at any point in time during the reproductive cycle, starting from ovulation and continuing through the first trimester of pregnancy, to inhibit pregnancy and cause menstruation. A composition of this invention is advantageously used in early pregnancy, when menses is delayed, or prior to the occurrence of menses. A composition of the present invention is preferably administered up to the eighth week of pregnancy, to inhibit corpus luteum function and, effectively, terminate pregnancy, just prior to expected menstruation, or when menstruation is delayed by up to 90 days. Such a composition is often less efficacious in late pregnancy, particularly in the third trimester.

The effective total dosage of the first and second prostaglandins depends on the route of administration. When administered intramuscularly, the effective total dosage is generally from 0.1 to 50 mg/kg body weight. Equivalent dosages may be employed for alternative routes of administration. The "total dosage" refers to the total amount of the first and second prostaglandins employed to achieve the desired result. This total dosage may be administered all at once, e.g. as a single injection, or over a short time period, e.g. by injection every eight hours for several days.

The weight ratio of the first and second prostaglandins in a composition of the invention is usually dependent on the particular prostaglandins. As for the total dosage, this factor may be determined by a skilled physician.

When 5-oxa-17-phenyl-17,19,20-trinor-$PGF_{1\alpha}$, or an alkyl ester thereof, and 9-deoxy-16,16-dimethyl-9-methylene-$PGE_2$, or an alkyl ester thereof, are employed, the weight ratio of the $PGF_{1\alpha}$ compound to the $PGE_2$ compound is preferably from 2:1 to 20:1. When $PGF_{2\alpha}$, 1,15-lactone is employed, the weight ratio of the $PGF_{1\alpha}$ compound to the lactone compound is preferably from 2:1 to 5:1. When the first prostaglandin is 5-oxa-17-phenyl-18,19,20-trinor-$PGF_{1\alpha}$, amide and the second 9-deoxy-16,16-dimethyl-9-methylene-$PGE_2$, or an alkyl ester thereof, the weight ratio of the amide to the $PGE_2$ compound is preferably from 5:1 to 30:1.

A composition of the invention may be in the form of a pharmaceutical composition comprising, in addition to the first and second prostaglandins, a physiologically acceptable excipient. The composition may be in a sterile form, suitable for intravenous infusion or subcutaneous or intramuscular injection. Other routes of administration for which the compositions may suitably be prepared are nasal, oral, buccal, intravaginal, intracervical, intrauterine and rectal. The compositions may be formulated into slow-release vehicles or polymers, such as silicone rubber, to form devices for subcutaneous, intravaginal, intracervical or intrauterine administration.

The use of a novel composition may provide results superior to the administration of one of the prostaglandins administered individually. It is of course highly desirable that a menses-inducing agent should be 100% effective. It is possible to formulate a composition of the invention which can provide 100% pregnancy inhibition at a lower dosage than is required for either prostaglandin administered individually.

The lower dosages which the compositions of the present invention can involve significantly increases the safety of their use. Side effects, such as nausea, fever and diarrhoea, can be decreased. By comparison with prostaglandins of the E series which are known for their thermogenic effect at dosages employed for menses induction, such effects can be avoided by using the combination compositions of the present invention.

As is noted above, a problem associated with finding menses-inducing agents for primates, including humans, is that agents which exhibit good luteolytic activity in rodents such as rats and hamsters, are frequently ineffective in inducing menses in primates.

Thus, 9-deoxo-16,16-dimethyl-9-methylene-$PGE_2$ by itself is not very effective as an antifertility agent in hamsters, but is very effective in combination with 5-oxa-17-phenyl-18,19,20-trinor-$PGF_{1\alpha}$, methyl ester for menses induction, as seen by the example below.

While all of the combinations disclosed herein are virtually 100% effective in inhibiting pregnancy in primates at the effective dose, the combination using 5-oxa-17-phenyl-18,19,20-trinor-$PGF_{1\alpha}$, methyl ester and 9-deoxo-16,16-dimethyl-9-methylene-$PGE_2$ is most preferred.

Pharmacologically acceptable salts of the formulas IV and V compounds useful for the purposes

described above are those with pharmacologically acceptable metal cations, ammonium, amine cations, or quaternary ammonium cations.

Especially preferred metal cations are those derived from the alkali metals, e.g. lithium, sodium, and potassium, and from the alkaline earth metals, e.g., magnesium and calcium, although cationic forms of other metals, e.g., aluminium, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, $\alpha$-phenylethylamine, $\beta$-phenylethylamine, ethylenediamine, diethylenetriamine, and like aliphatic, cycloaliphatic and araliphatic amines containing up to and including 18 carbon atoms, as well as heterocyclic amines, e.g., piperidine, morpholine, pyrrolidine, piperazine, and lower-alkyl derivatives thereof, e.g., 1-methylpiperidine, 4-ethylmorpholine, 1-isopropylpyrrolidine, 2-methylpyrrolidine, 1,4-dimethylpiperazine and 2-methylpiperidine, as well as amines containing water-solubilizing or hydrophilic groups, e.g., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl)aminomethane, N-(p-tert-amylphenyl)diethanolamine, galactamine, N-methylglycamine, N-methylglucosamine, ephedrine, phenylephrine, epinephrine and procaine.

Examples of suitable pharmacologically acceptable quarternary ammonium cations are tetramethylammonium, tetraethylammonium, benzyltrimethylammonium and phenyltriethylammonium.

The invention is illustrated by the following Examples. Reference Examples are also given.

## Example 1

Three female rhesus monkeys were placed with males from days 11 to 15 of the menstrual cycle. 5 ml blood samples were collected daily between 7 and 9:00 a.m. beginning on day 20 of the menstrual cycle and continued until day 36 from the previous menses. Concentrations of monkey chorionic gonadotropin (mCG) and progesterone were determined for each blood sample by radioimmunoassay.

Pregnancy was confirmed in all monkeys prior to treatment by the qualitative determination of mCG in the serum. Treatments were given by intramuscular injection at 7:00 a.m., 3:00 p.m. and 11:00 p.m. of day 28 from the previous menses. Prostaglandins were given as an emulsion in 1 ml of 4% glass-distilled ethanol and 96% sterile aqueous vehicle, containing 10 mg of carboxymethylcellulose, 4 mg of polysorbate 80, and 0.42 mg of propylparaben per milliter. 75 mg of 5-oxa-17-phenyl-18,19,20-trinor-$PGF_{1\alpha}$, methyl ester and 0.5 mg of 9-deoxo-16,16-dimethyl-9-methylene-$PGE_2$ were administered at each injection. The prostaglandins were mixed together in the same vehicle and given at the same injection site.

Pregnancy terminated promptly in the three monkeys given the combination of prostaglandins. Vaginal bleeding commenced on the day after treatment in two of these monkeys, and vaginal bleeding began on the second day following prostaglandin administration in the third monkey.

Serum progesterone and mCG declined to 10% of pretreatment values within 24 hours of initial treatment. Progesterone remained depressed throughout the study, and mCG disappeared from the blood.

Slight anorexia was observed in one of the monkeys on the day of treatment.

## Reference Example A

The procedure of Example 1 was followed exactly using another three monkeys, except that no 5-oxa-17-phenyl-18,19,20-trinor-$PGF_{1\alpha}$, methyl ester was administered. Pregnancy terminated in one of these monkeys, after a delay of 4 weeks from treatment.

## Example 2

Following the procedure of Example 1, three pregnant rhesus monkeys were given a single injection of 7.5 mg of 5-oxa-17-phenyl-18,19,20-trinor-$PGF_{1\alpha}$, methyl ester and 0.5 mg of 9-deoxo-16,16-dimethyl-9-methylene-$PGE_2$ at 7:00 a.m. on day 28 of the menstrual cycle. Progesterone and mCG dropped to 10% of pretreatment levels within 24 hours of injection. Pregnancy terminated promptly in all three monkeys; menstrual bleeding was first observed on the day of treatment, the day after treatment, and 8 days after treatment, respectively for the three monkeys. No side-effects were observed in any of the monkeys.

## Example 3

Using the procedure of Example 1, three rhesus monkeys were given three doses of 5 mg of 5-oxa-17-phenyl-18,19,20-trinor-$PGF_{1\alpha}$, methyl ester and 1 mg of $PGF_{2\alpha}$,1,15-lactone. The prostaglandins were mixed together in the same vehicle and given at the same injection site.

Pregnancy terminated in all three monkeys, promptly in two. For these monkeys, progesterone and mCG declined markedly within 24 hours of treatment and remained depressed throughout the study. Serum progesterone and mCG declined in the third monkey and remained depressed throughout the study. This monkey was no longer pregnant 18 days after blood sampling was completed.

Slight appetite depression was observed on the day of treatment of all three monkeys. The animals ate approximately two-thirds of the food provided for them, but no other side-effects were seen.

Reference Example B

Following the procedure of Example 1, 7.5 mg of 5-oxa-17-phenyl-18,19,20-trinor-$PGF_{1\alpha}$, methyl ester in one ml of sterile vehicle, were injected on days 28 and 29 of the menstrual cycle to each of two monkeys, and on days 27 and 28 for one monkey, at 7:00 a.m. and 7:00 p.m. Another 3 monkeys were injected with sterile vehicle only, following the same regimen, on days 28 and 29.

Pregnancy terminated in one of the monkeys receiving the prostaglandin compound. Serum progesterone declined below 1 ng/ml within 24 hours of initial treatment and remained depressed throughout the study. Serum progesterone declined to 75% of normal in the other two treated monkeys within 24 hours of initial treatment, but rebounded to normal despite continued treatment.

Slight diarrhoea was observed in two of the treated monkeys. None of the three ate their food on days of treatment.

Reference Example C

Following the procedure of Example 1, nine 7.5 mg injections of 5-oxa-17-phenyl-18,19,20-trinor-$PGF_{1\alpha}$, methyl ester were administered to each of two pregnant monkeys. The total dosage was 67.5 mg per monkey. Pregnancy terminated in one monkey. The monkeys experienced slight appetite depression on the days of treatment.

Reference Example D

Using the procedure of Reference Example B, two injections of $PGF_{2\alpha}$, 1,15-lactone were given on day 28 of the menstrual cycle in 3 mg portions to each of three female monkeys. Pregnancy was terminated in one of the three animals.

Example 4

Nine female rhesus monkeys of proven fertility were caged with male rhesus monkeys of proven fertility from days 11 to 15 of the menstrual cycle. The female monkeys received intramuscular injections of 1.5 mg of 5-oxa-17-phenyl-18,19,20-trinor-$PGF_{1\alpha}$, methyl ester and 0.1 mg of 9-deoxo-16,16-dimethyl-9-methylene-$PGF_2$ once daily on days 18 to 22 of the menstrual cycle. The prostaglandins were mixed together in the same vehicle and given at the same injection site. None of the monkeys treated with the combination became pregnant.

Example 5

Following the procedure of Example 1, three injections, each containing 7.5 mg of 5-oxa-17-phenyl-18,19,20-trinor-$PGF_{1\alpha}$, amide and 0.5 mg of 9-deoxy-9-methylene-16,16-dimethyl-$PGE_2$, were administered to two pregnant rhesus monkeys, in eight hour intervals on day 28 of the menstrual cycle. Pregnancy terminated in both monkeys.

**Claims**

1. A pharmaceutical composition comprising a first prostaglandin of the formula

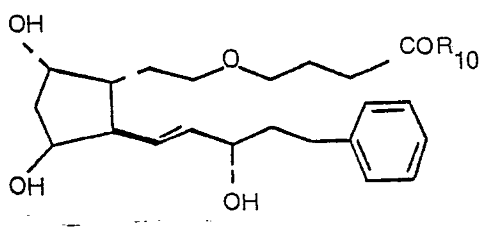

wherein $R_{10}$ is $NH_2$, OH, $C_{1-12}$ alkoxy or OM and M is a pharmacologically acceptable cation, and a second prostaglandin which is $PGF_{2\alpha}$, 1,15-lactone or is of the formula

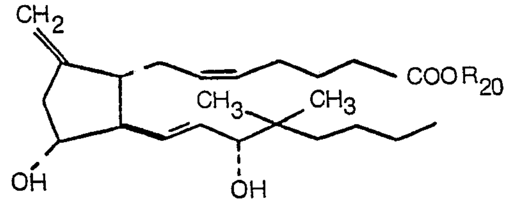

wherein $R_{20}$ is hydrogen, $C_{1-12}$ alkyl or a pharmacologically acceptable cation.

2. A composition according to claim 1, wherein the first prostaglandin is the methyl ester or the amide of 5-oxa-17-phenyl-18,19,20-trinor-$PGF_{1\alpha}$.

3. A composition according to claim 1 or claim 2, wherein the second prostaglandin is 9-deoxo-16,16-dimethyl-9-methylene-$PGE_2$ or $PGF_{2\alpha}$, 1,15-lactone.

4. A composition according to any preceding claim, which additionally comprises a physiologically acceptable excipient.

5. A composition according to any preceding claim, for inducing menses in a female primate.

## Revendications

1. Composition pharmaceutique comprenant une première prostaglandine de formule

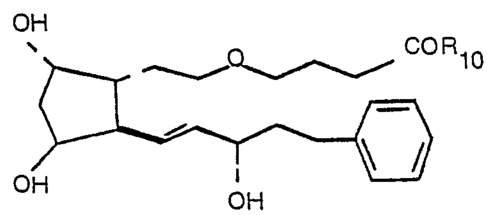

dans laquelle $R_{10}$ représente $NH_2$, OH, un groupe alkoxy en $C_1$ à $C_{12}$ ou un groupe OM, et M est un cation pharmacologiquement acceptable, et une seconde prostaglandine qui est la 1,15-lactone de $PGF_{2\alpha}$ ou qui répond à la formule

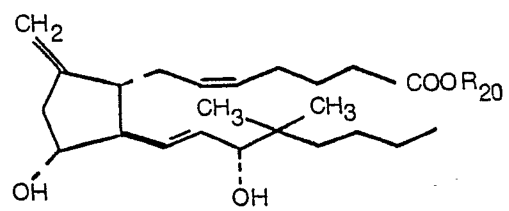

dans laquelle $R_{20}$ est l'hydrogène, un groupe alkyle en $C_1$ à $C_{12}$ ou un cation pharmacologiquement acceptable.

2. Composition suivant la revendication 1, dans laquelle la première prostaglandine est l'ester méthylique ou l'amide de 5-oxa-17-phényl-18,19,20-trinor-$PGF_{1\alpha}$.

3. Composition suivant la revendication 1 ou la revendication 2, dans laquelle la seconde prostaglandine est la 9-déoxo-16,16-diméthyl-9-méthylène-$PGE_2$ ou la 1,15-lactone de $PGF_{2\alpha}$.

4. Composition suivant l'une quelconque des revendications précédentes, qui comprend en outre un excipient physiologiquement acceptable.

5. Composition suivant l'une quelconque des revendications précédentes, destinée à induire la menstruation chez une femelle de primate.

## Patentansprüche

1. Eine pharmazeutische Zusammensetzung, dadurch gekennzeichnet, dass sie ein erstes Prostaglandin der Formel

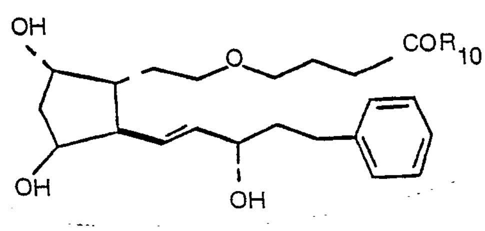

worin $R_{10}$ $NH_2$, OH, $C_{1-12}$ Alkoxy oder OM bedeutet, worin M ein pharmakologisch annehmbares Kation ist, und ein zweites Prostaglandin, welches $PGF_{2\alpha}$, 1,15-Lacton ist oder die Formel

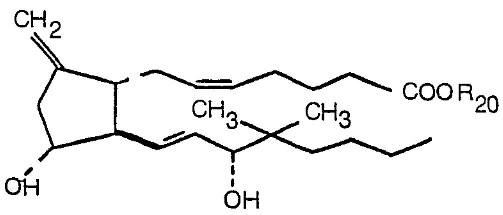

aufweist, worin $R_{20}$ Wasserstoff, $C_{1-12}$ Alkyl oder ein pharmakologisch annehmbares Kation bedeutet, enthält.

2. Zusammensetzung nach Anspruch 1, dadurch gekennzeichnet, dass das erste Prostaglandin der Methylester oder das Amid von 5-Oxa-17-phenyl-18,19,20-trinor-$PGF_{1\alpha}$ ist.

3. Zusammensetzung nach Anspruch 1 oder 2, dadurch gekennzeichnet, dass das zweite Prostaglandin 9-Desoxo-16,16-dimethyl-9-methylen-$PGE_2$ oder $PGF_{2\alpha}$, 1,15-Lacton ist.

4. Zusammensetzung nach irgendeinem der vorangegangenen Ansprüche, dadurch gekennzeichnet, dass sie zusätzlich einen physiologisch annehmbaren Arzneimittelträger enthält.

5. Zusammensetzung nach irgendeinem der vorangegangenen Ansprüche für die Herbeiführung der Monatsblutung in einem weiblichen Primaten.